Europäisches Patentamt

European Patent Office

Office européen des brevets

(19)

(11) EP 1 386 616 A1

(12) DEMANDE DE BREVET EUROPEEN

(43) Date de publication:
04.02.2004 Bulletin 2004/06

(51) Int Cl.7: A61K 39/10, C07K 14/235,
C12N 1/20, C07K 16/12

(21) Numéro de dépôt: 03010630.6

(22) Date de dépôt: 15.12.1995

(84) Etats contractants désignés:
AT BE CH DE DK ES FR GB IE IT LI NL SE

(30) Priorité: 15.12.1994 FR 9415137

(62) Numéro(s) de document de la (des) demande(s) initiale(s) en application de l'article 76 CBE:
95402838.7 / 0 719 560

(71) Demandeur: INSTITUT PASTEUR
75724 Paris Cédex 15 (FR)

(72) Inventeurs:
• Gueirard, Pascale
75724 Paris Cedex 15 (FR)

• Guiso, Nicole
75013 Paris (FR)

(74) Mandataire: Desaix, Anne et al
Ernest Gutmann - Yves Plasseraud S.A.
3, rue Chauveau-Lagarde
75008 Paris (FR)

Remarques:
This application was filed on 12 - 05 - 2003 as a divisional application to the application mentioned under INID code 62.

(54) **Vaccin anti-bordetella**

(57)    L'invention concerne une composition immunogène, caractérisée en ce qu'elle comprend une protéine adénylcyclase-hémolysine (AC-Hly) ou une partie immunogène de cette AC-Hly, d'une souche de Bordetella choisie parmi B. pertussis, B. parapertussis ou B. bronchiseptica et en ce qu'elle comprend en outre un extrait bactérien contenant les produits d'expression des gènes vrg d'une souche de Bordetella choisie parmi B. pertussis, B. parapertussis ou B. bronchiseptica ou une partie de ces produits d'expression, suffisante pour induire une réponse immunitaire chez un hôte auquel l'extrait serait administré.

EP 1 386 616 A1

## Description

Le genre Bordetella comprend quatre espèces Bordetella pertussis, Bordetella parapertussis, Bordetella bronchiseptica et Bordetella avium.

Les Bordetellae sont des coccobacilles gram-négatif responsables d'infections respiratoires. Bordetella pertussis et Bordetella parapertussis, agents de la coqueluche, sont des pathogènes strictement humains. Bordetella bronchiseptica est pathogène pour divers mammifères et, plus rarement pour l'homme et à la différence de B. pertussis et B. parapertussis est capable de survivre à l'extérieur de l'hôte. Bordetella avium n'est pathogène que pour l'oiseau.

Depuis l'introduction de la vaccination coquelucheuse dans les pays où la couverture vaccinale est supérieure à 80%, on a pu observer une chute spectaculaire de la morbidité et de la mortalité. Cette chute est bien attribuable à la vaccination puisque dans plusieurs pays (Grande-Bretagne, Suède, Japon...) des épidémies meurtrières de coqueluche ont eu lieu dans les années qui ont suivi l'arrêt de la vaccination.

L'invention propose des compositions immunogènes pouvant entrer dans la constitution de vaccins coquelucheux, ces compositions étant au moins en partie de type "acellulaire" et présentant une efficacité au moins identique à celle du vaccin connu.

L'invention concerne d'une part des vaccins utilisables en médecine vétérinaire et d'autre part des vaccins utilisables en médecine humaine.

Le vaccin coquelucheux utilisé actuellement est un vaccin cellulaire, composé de suspensions bactériennes de B. pertussis (mélange de deux souches différant dans l'expression d'agglutinogènes) inactivées par chauffage. Ce vaccin est généralement utilisé sous forme combinée avec des fractions purifiées diphtérique et tétanique, le composant haemophilus et le composant viral polio inactivé. La vaccination consiste en trois injections à un mois d'intervalle dès l'âge de deux mois et une injection à 18 mois. Aucune autre injection de rappel n'est ensuite pratiquée.

ce vaccin est parfois mal toléré, tant localement que généralement. Il a, en particulier, été accusé d'entraîner des complications neurologiques graves à type d'encéphalite aigüe ; cependant, des études très récentes semblent conclure à l'absence de preuve statistique d'une relation entre le vaccin cellulaire et les complications neurologiques sévères (Griffiths AH. Vaccine 1989 ; 7:199-210).

Il n'en demeure pas moins vrai que le vaccin cellulaire est mal toléré et est responsable d'effets, réversibles mais indésirables. Pour ces raisons, un nouveau vaccin dépourvu de ces effets est souhaitable. Pour envisager la définition d'un nouveau vaccin, il apparaissait nécessaire de caractériser certains facteurs impliqués dans la virulence de la bactérie et le cas échéant dans la régulation de la virulence. Purifiés, ces différents facteurs sont autant de candidats théoriques pour la constitution d'un vaccin coquelucheux dit "acellulaire" par opposition au vaccin classique. Ce nouveau type de vaccin devrait apporter, en plus d'une meilleure tolérance, une efficacité au moins égale à celle du vaccin classique.

Les facteurs impliqués dans la virulence de B. pertussis ont été identifiés comme suit : la coqueluche peut être schématiquement définie par l'association d'un syndrome infectieux, impliquant l'adhésion des bactéries aux cellules cibles (cellules ciliées de l'appareil respiratoire), sans invasion ni dissémination dans l'organisme de l'hôte et d'un syndrome toxique secondaire incluant des effets cytopathogènes locaux, électifs pour l'épithélium respiratoire cilié (destruction et élimination des cellules ciliées, accumulation du mucus, réaction inflammatoire) et des effets systémiques dont le plus évident est l'hyperleucocytose avec hyperlymphocytose.

Grâce aux techniques récentes de biologie moléculaire, un certain nombre de facteurs impliqués dans la virulence de B. pertussis ont été caractérisés et la régulation de leur expression connue. Ces facteurs peuvent être classés en deux catégories, ceux participant au syndrome infectieux (adhésines) et ceux jouant un rôle dans le syndrome toxinique (toxines).

## Les adhésines:

- l'hémagglutinine filamenteuse ou **FHA** est considérée comme jouant un rôle majeur dans l'adhésion de la bactérie sur l'épithélium cilié (Locht C., Bertin P., Menozzi F. D. and Renaud G. Mol. Microbiol. 1993, 9:653-66). La FHA est toujours exprimée par les souches virulentes et est sécrétée. Son gène de structure a été cloné et séquencé (Relman D. et al, 1989, Proc. Natl. Acad. Sci. USA, 86:2637-2641). Il code pour une protéine de 360 kDa, mais seul un fragment de 220 kDa peut être purifié. Cette protéine se fixe aux glycoprotéines des cellules ciliées et elle possède des sites de fixation pour les intégrines des lymphocytes et des macrophages. Il vient d'être montré récemment que la FHA présente une homologie avec certaines protéines des cellules endothéliales de l'hôte (Tuomanen E., Prasad S. M., George J. S., Hoepelman A. I. M., Ibsen P., Heron I., and Starzyk R. M. 1993. Proc. Natl. Sci. USA. 90:7824-7828).

- Les deux agglutinogènes ou **AGG** de B. pertussis permettent de classer les souches en sérotypes. Deux AGG ont été caractérisés. Ces protéines sont sécrétées et jouent un rôle dans l'adhésion de la bactérie aux cellules épithéliales (Mooi F. Van der Heide H. G. D., Ter Avest A. R., Welinder K.G., Livey I., Van der Zeijst B.A.M., and Gaastra, W. 1987. Microb. Pathog. 2:473-484).

- La pertactine ou **PRN,** est une protéine de 93 kDa, mais seul un fragment de 69 kDa peut être purifié. Cette protéine possède deux sites de fixation pour les intégrines des macrophages et des lymphocytes (Charles I, Dougan G., Pickard D., Chattfield S. Smith M. Novotny P., Morissey P. and Fairweather N.F. 1989 Proc Natl Acad Sci. 86: 3554-3558).

- La toxine de pertussis ou **PTX,** toxine de type A-B, sécrétée qui, outre ses effets cytopathogènes, participe à l'adhésion par l'intermédiaire de sa sous-unité B. L'oligomère B est capable de se fixer sur les récepteurs des cellules ciliées mais pas nécessairement sur les mêmes récepteurs que ceux de la FHA. La fixation de la PTX sur les leucocytes empêcherait leur migration vers le site de la réaction inflammatoire. cette fixation induirait une augmentation du nombre de molécules d'intégrines fonctionnelles sur les leucocytes ce qui favoriserait la fixation de la bactérie par l'intermédiaire de la FHA (Rozindski E., Burnette W.N., Jones T., Mar V., and Tuomanen E. 1993 J. Exp. Med. 178:917-924).

**Les toxines:**

- La toxine de pertussis ou **PTX** est sécrétée et considérée comme la toxine majeure de B. pertussis. Sa **sous-unité A**, possède une activité ADP-ribosyl transférase. Après fixation de la partie B de la toxine sur la cellule cible, cette sous-unité A est capable de pénétrer dans la cellule, d'inactiver les protéines régulatrices G et d'entraîner ainsi une perturbation de toutes les fonctions cellulaires. c'est ce facteur qui serait responsable des effets biologiques généralisés observés lors de la maladie tels l'hyperlymphocytose, l'hyperinsulinémie, la sensibilité à l'histamine.

- La toxine dermonécrotique ou **TDN**, non encore bien caractérisée et la toxine cytotrachéale ou **TCT**, petite glycoprotéine sécrétée de la famille des muramyl peptides, dérivée du peptidoglycane de la bactérie, agiraient de concert pour détruire les cellules ciliées de l'appareil respiratoire de l'hôte. La TCT empêche de plus la régénération de l'épithélium respiratoire (Luker K., Collier J. L., Kolodziej E. W., Marshall G.R., and Goldman W.E. 1993. Proc. Natl. Acad. Sci. USA. 90:2365-2369).

- L'adényl cyclase-hémolysine ou **AC-Hly**, est une protéine bifonctionnelle, possédant une activité adényl cyclase et une activité hémolytique. Elle est sécrétée par la bactérie. Son gène de structure a été cloné et séquencé (Glaser P. et al, 1988, Molec. Microb. 2, 19-20). Il s'avère que cette protéine fait partie de la famille des toxines dites "RTX" pour "repeats in toxins" et présente des homologies avec l'hémolysine d'Escherichia coli, d'Actinobacillus pleuropneumoniae, les leucotoxines de Pasteurella haemolytica et d'Actinobacillus actinomycetemcomitans. Cette protéine, comme la PTX, est capable de pénétrer dans les cellules eucaryotes telles que les macrophages, d'être activée par la calmoduline, de synthétiser de grandes quantités d'AMPc et de perturber les fonctions cellulaires (Coote J. 1992. FEMS Microbiol. Rev. 88:137-162).

De même, les facteurs impliqués dans la virulence de B. parapertussis et B. bronchiseptica ont été identifiés.

Les infections à B. pertussis, B. parapertussis et B. bronchiseptica sont indistinguables d'un point de vue clinique. Ces bactéries ont plus de 75% d'homologie au niveau de l'ADN. Elles n'ont été classifiées en espèces que sur la base de caractères phénotypiques et biochimiques. B. parapertussis et B. bronchiseptica synthétisent des facteurs de virulence très proches fonctionnellement et immunologiquement de B. pertussis à l'exception de la PTX.

Un vaccin composé de suspensions bactériennes de B. pertussis inactivées protège contre une infection à B. pertussis mais aussi contre une infection à B. parapertussis et à B. bronchiseptica dans le modèle murin. Bien qu'il n'y ait pas de données épidémiologiques sur les infections à B. parapertussis en France, il est à noter que l'on isole peu de souches de cette espèce dans notre pays, pays vacciné depuis 25 ans avec un "vaccin pertussis", alors qu'elles sont isolées dans les pays non ou mal vaccinés.

Outre la présence de ces différentes adhésines et toxines, les Bordetelles sont caractérisées par une régulation de l'expression des facteurs impliqués dans leur virulence. Autrement dit, les Bordetelles subissent des variations et modulations de phase.

Les Bordetelles, suivant leur environnement, peuvent devenir "avirulentes" c'est à dire incapables d'induire la létalité, une réaction inflammatoire et des lésions pulmonaires dans le modèle murin d'infection respiratoire. Elles subissent soit une modulation de phase soit une variation de phase. La variation de phase s'observe à une fréquence allant de $10^{-3}$ à $10^{-6}$ et est quasiment irréversible. Elle se traduit par un arrêt de l'expression des toxines et adhésines décrites précédemment et par l'expression d'autres facteurs non encore bien caractérisés (changement des bactéries "virulentes" Phase I en bactéries "avirulentes" Phase IV). Les bactéries de phase I et de phase IV ont été décrites par Lacey B. 1960, J. Hyg, 58:57-93. La modulation de phase, phénotypiquement semblable à la variation de phase est totalement réversible et se traduit par un arrêt de la synthèse des adhésines et des toxines lors de changements environnementaux (composition du milieu de culture, température...).

La variation de phase et la modulation de phase observées chez Bordetella sont sous le contrôle de deux gènes régulateurs, bvg A et bvg S (Arico B. et al, 1989, Proc. Natl. Acad. Sci USA, 86: 6671-6675).

Le gène bvg S code pour une protéine sensible aux conditions extérieures. Cette protéine module par phosphorylation l'activité de la protéine codée par le gène bvgA, qui est d'une part un activateur positif de la transcription des gènes codant pour les facteurs de virulence (gènes vag pour "vir activated genes") cités précédemment (Uhl M. A. And Miller J. 1994. PRoc. Natl. Acad. Sci USA 91:1163-1167), d'autre part un répresseur de la transcription de certains gènes (Beattie D.T. et al, J. of Bacteriology, Jan 93, p. 159-527). Les gènes dont l'expression est réprimée sont appelés gènes vrg pour "vir repressed genes" et sont encore mal caractérisés. Il a cependant été montré que le gène vrg 6 de B. pertussis code pour une protéine ayant un rôle dans la persistance de la bactérie chez l'hôte (Beatties D. et al, 1992, Infect. Imm. 60:571-577). Chez B. bronchiseptica, deux protéines codées par les gènes vrg ont été caractérisées : ce sont des protéines de type flagelles (B. bronchiseptica Phase I est une bactérie immobile ne synthétisant pas de flagelles mais synthétisant adhésines et toxines et B. bronchiseptica Phase IV est une bactérie mobile synthétisant des flagelles).

Afin de mesurer la virulence des bactéries et d'évaluer les effets toxiniques locaux et généraux, un modèle d'infection respiratoire de la souris a été mis au point (Guiso N. et al, 1991, Microb. Pathogen 11, 423-431). A l'aide de ce modèle murin, il a pu être montré que la PTX inactivée chimiquement ou génétiquement, est un bon immunogène. Cette anatoxine a une activité protectrice contre des infections létales à B. pertussis, mais ne semble pas induire la synthèse d'anticorps efficaces contre la persistance de la bactérie. (Khelef. N. Danve B. Quentin-Millet M. J and Guiso N. 1993 Infect Immun. 64:486-490).

Ces résultats relatifs à la virulence des Bordetelles et à la régulation de cette virulence, montrent que la coqueluche est une maladie multifactorielle et que le vaccin doit non seulement protéger contre des infections létales mais aussi contre la persistance de la bactérie. Des conclusions similaires s'appliquent aux infections dues à B. parapertussis ou à B. bronchiseptica.

Des tentatives de développement d'un vaccin acellulaire, à partir des composants isolés de la famille des adhésines ou des toxines ont été effectuées. Ainsi, des compositions acellulaires contenant soit la toxine de B. pertussis (PTX) purifiée soit cette toxine associée à l'hémagglutinine filamenteuse (FHA) purifiée ont été préparées.

Les premiers essais de tolérance chez l'homme de ces compositions acellulaires (PTX ou PTX-FHA) montrent une nette diminution des complications tant locales (douleur, gonflement) que générales (fièvre, convulsions..) en comparaison avec le vaccins cellulaire classique (Edward K., J. Infect. Dis. 1993, 168, 15-20).

Ces nouvelles préparations (PTX ou PTX-FHA) ont une bonne immunogénicité et induisent un taux élevé d'anticorps. Cependant, la recherche d'anticorps vaccinaux est une méthode imparfaite car une séroconversion n'est pas synonyme de protection contre la maladie et aucune démonstration n'a été faite du caractère protecteur des anticorps obtenus, ni du taux éventuel de protection.

Les résultats d'essais cliniques de différents vaccins cellulaires et acellulaires ont été publiés (International Symposium on Pertussis Vaccine trials, Rome 30.10.95-1.11.95). Ces résultats montrent que tous les vaccins cellulaires ne sont pas équivalents, certains sont très efficaces et induisent peu d'effets secondaires et d'autres sont très peu efficaces et induisent des effets secondaires plus importants.

Les résultats publiés montrent que les vaccins acellulaires testés, monovalent (PTX), bivalent (PTX, FHA), trivalent (PTX, FHA, PRN) ou pentavalent (PTX, FHA, PRN, AGG2, AGG3) induisent très peu d'effets secondaires, sont tous immunogènes et ont tous une efficacité contre la maladie (selon la définition OMS) supérieure ou égale à 70%. Cependant, l'efficacité d'un vaccin acellulaire PTX-FHA est toujours inférieure quelle que soit la définition de cas, à celle d'un vaccin cellulaire efficace.

Malgré les résultats encourageants obtenus s'agissant de l'immunogénicité de différentes compositions contenant à la fois des adhésines et des toxines de B. pertussis, les inventeurs ont estimé qu'une protection efficace contre la maladie due à une infection par B. pertussis, B. parapertussis ou B. bronchiseptica, nécessitait de considérer des facteurs supplémentaires par rapport aux adhésines et toxines et en particulier des facteurs intervenant dans la persistance de la bactérie.

Leurs observations les ont conduits à définir un modèle décrit en détail dans la partie expérimentale, à partir duquel de nouveaux critères préalables à la définition de vaccins ont été définis.

Ainsi, selon la présente demande, pour définir des vaccins efficaces et substantiellement non toxiques, contre l'une au moins des Bordetelles B. pertussis, B. parapertussis ou B. bronchiseptica, il convient de mettre en oeuvre non seulement une ou plusieurs adhésines et/ou toxines de ces bactéries, mais également un ou plusieurs facteurs dont la synthèse est réprimée lorsqu'il y a expression des toxines et adhésines de la bactérie. Ces facteurs sont notamment des produits d'expression des gènes vrg dont il a été question précédemment.

Dans le but de préparer un vaccin coquelucheux dépourvu d'effets secondaires et protecteur contre les effets locaux ou systémiques dus aux toxines synthétisées par B. pertussis et/ou B. parapertussis et/ou B. bronchiseptica, et protecteur contre la persistance chez l'hôte de ces bactéries, l'invention a pour objet une composition immunogène, caractérisée en ce qu'elle comprend une protéine adénylcyclase-hémolysine (AC-Hly) ou une partie immunogène de cette AC-Hly, caractéristique d'une souche de Bordetelle choisie parmi B. pertussis, B. parapertussis ou B. bronchiseptica et en ce qu'elle comprend en outre un extrait bactérien contenant les produits des gènes vrg d'une souche de Bordetelle choisie parmi B. pertussis, B. parapertussis ou B. bronchiseptica ou une partie de ces produits d'expression, suffisante pour induire une réponse immunitaire chez un hôte auquel l'extrait serait administré.

Aucun des vaccins acellulaires élaborés jusqu'à ce jour ne contient d'AC-Hly. Or, selon les inventeurs, cette protéine devrait être incorporée dans une composition vaccinante dès lors qu'elle joue un rôle important dans la virulence des Bordetelles pour les raisons suivantes :

*    elle est exprimée très précocément après l'infection puisque des anticorps spécifiques sont synthétisés et détectés, aussi bien chez l'homme infecté que chez l'animal infecté, dès le début de l'infection (Khelef N, Sakamoto H., and Guiso N 1992. Microbiol. Pathogen. 12:227-235 et Gueirad P. and Guiso N. 1993 Infect. Immun. 61:4072-4078).
*    elle est nécessaire à la bactérie pour initier l'infection (Khelef N. Sakamoto H. and Guiso N. 1992 Microbiol. Pathogen. 12:227-235).
*    son utilisation sous forme purifiée comme antigène dans le modèle murin d'infection respiratoire protège les souris contre la colonisation de l'appareil respiratoire (Guiso , Szatanik, et Rocancourt . 1989 Microb. Pathogen. 11: 423-431).
*    elle est responsable, in vitro, de la mort des macrophages alvéolaires par apoptose (Khelef, Zyglinski, et Guiso Infect. Immun. 61:4064-4071). Les autres adhésines ou toxines n'interviennent pas dans le processus d'apoptose.

Par ailleurs, la présence dans la composition immunogène, d'un extrait bactérien comprenant les produits d'expression des gènes vrg permettrait d'améliorer la réponse immunitaire humorale et/ou cellulaire obtenue après infection chez les sujets vaccinés et contribuerait également à la protection contre la persistance de la bactérie.

L'extrait bactérien dit "extrait bactérien vrg" dont question ci-dessus contient tous les constituants de la membrane externe, y compris l'endotoxine LPS, d'une bactérie de phase IV, c'est à dire d'une bactérie n'exprimant pas les gènes vag. L'endotoxine LPS peut alternativement être éliminée ou détoxifiée.

Cet extrait peut être présent sous forme de suspension.

Une première composition immunogène préférée de l'invention est une composition immunogène caractérisée en ce qu'elle comprend en outre une ou plusieurs adhésines ou toxines respectivement de B. pertussis, B. parapertussis ou B. bronchiseptica, choisie(s) parmi la FHA, les AGG ou la PRN et la PTX.

Les adhésines des Bordetella renforcent le caractère immunogène de la composition contenant la AC-Hly et l'extrait bactérien vrg.

Selon un premier mode de réalisation de l'invention, la composition immunogène est caractérisée en ce qu'elle comprend la toxine PTX de B. pertussis, la toxine AC-Hly de B. pertussis et les adhésines FHA et PRN et un extrait bactérien contenant les protéines codées par les gènes vrg de B. pertussis ou une partie de ces protéines, suffisante pour induire une réponse immunitaire chez un hôte auquel l'extrait serait administré.

Une telle composition peut être mise en oeuvre pour préparer des vaccins protecteurs contre les effets létaux et systémiques de B. pertussis chez l'homme et contre la persistance de la bactérie.

Selon un mode de réalisation préféré de l'invention, la toxine AC-Hly de B. pertussis est également contenue dans un extrait bactérien contenant tout ou partie des facteurs de virulence du groupe des adhésines ou des toxines de B. pertussis.

Les extaits bactériens "vag" ou "vrg" auxquels il est fait appel pour réaliser l'invention, sont de préférence des extraits dits "extraits urée".

Un "extrait urée" est composé d'un mélange de protéines exprimées à la surface de la bactérie et qui sont séparées de la bactérie après incubation de celle-ci avec de l'urée 5M. L'"extrait urée vag" de B. bronchiseptica par exemple contient entre autres protéines l'AC-Hly, la FHA, la PRN et le LPS (lipopolysaccharide endotoxine) et l'"extrait urée vrg" contient, plusieurs protéines non encore caractérisées, les flagelles et le LPS.

L'utilisation d'extraits urée permet notamment la réalisation d'un vaccin de moindre coût par rapport à un vaccin qui serait obtenu à partir des protéines contenues dans les extraits, sous forme purifiée.

En outre, les inventeurs ont constaté que les extraits urée utilisés peuvent induire une réponse immunitaire cellulaire de type T (lymphoprolifération), se comportant ainsi comme le vaccin cellulaire utilisé jusqu'à ce jour.

Au contraire, les compositions exclusivement acellulaires n'induiraient pas de réponse T, réaction qui se produit pourtant en cas d'infection.

Les extraits urée vag ou vrg sont préparés respectivement à partir des bactéries de phase I ou de phase IV. Le cas échéant, les bactéries de phase IV sont remplacées par des bactéries dont le gène bvgS est muté de façon telle que les bactéries n'expriment que les protéines codées par les gènes vrg.

La préparation de ces extraits est décrite en détail dans la partie expérimentale.

Ainsi, l'invention concerne de façon préférée, une composition immunogène comprenant à la fois un extrait urée vag de B. pertussis et un extrait urée vrg de B. pertussis.

Une souche de B. pertussis appropriée pour la préparation de ces extraits est la souche HAV entrant dans le cadre de l'invention et déposée à la CNCM (Collection Nationale de Cultures de Microorganismes à Paris), le 19 Octobre 1994 sous le n° I-1485. Pour préparer l'extrait urée vag, la souche HAV peut être utilisée directement puisqu'il s'agit d'une souche de phase I.

En revanche, l'extrait urée vrg est obtenu à partir d'une souche de phase IV dérivée de la souche de phase I par exemple par mutation du gène bvgs de la bactérie ou par culture de ladite souche de phase I dans un milieu contenant uniquement du sulfate de magnésium, de façon à obtenir l'expression des seuls gènes vrg de B. pertussis.

L'invention propose en outre des compositions immunogènes élaborées selon les principes décrits ci-dessus pour B. pertussis, à partir des Bordetelles de la famille de B. parapertussis ou de B. bronchiseptica.

Il est en effet connu que la vaccination avec le vaccin cellulaire disponible sur le marché, c'est à dire composé de suspensions bactériennes de bactéries de phase I de B. pertussis inactivées protège contre des infections à B. pertussis et à B. parapertussis dans le modèle murin. Cependant, il a été démontré récemment que l'administration des facteurs purifiés de B. pertussis (PTX ou FHA ou PRN) ne protège pas contre la maladie et l'infection dues à B. parapertussis. De même l'administration de l'AC-Hly purifiée de B. parapertussis ne protège pas contre une infection à B. pertussis dans le modèle murin.

Ces résultats suggèrent que malgré une homologie très élevée entre les deux espèces, la protection est spécifique d'espèce. L'utilisation de vaccins acellulaires, composés de PTX, PRN et FHA des seules souches B. pertussis, comme vaccins coquelucheux dans les années à venir, risquerait donc d'entraîner une augmentation des infections à B. parapertussis (Khelef Danve Quentin-Millet et Guiso 1993, Infect. Immun. 61:46-490 et Gueirad P., et Guiso 1993 Infect. Immun 61:4072-4078).

L'invention propose donc également des compositions immunogènes caractérisées en ce qu'elles comprennent la toxine AC-Hly de B. parapertussis et un extrait bactérien contenant tout ou partie des protéines codées par les gènes vrg de B. parapertussis.

Avantageusement, la toxine AC-Hly de B. parapertussis est contenue dans un extrait bactérien contenant tout ou partie des facteurs de virulence du groupe des adhésines ou des toxines de B. parapertussis. Un tel extrait est de préférence un extrait urée vag, obtenu par exemple à partir de B. parapertussis souche n° 1 déposée à la CNCM le 2 Décembre 1994, sous le n° I-1498. La souche de B. parapertussis n° I-1498 entre dans le cadre de la présente invention.

De même, l'extrait bactérien vrg est de préférence un extrait urée vrg obtenu par exemple à partir de B. parapertussis souche n° 1 déposée à la CNCM le 2 Décembre 1994, sous le n° I-1498, selon des modalités analogues à celles qui ont été décrites pour la souche B. pertussis.

Selon un autre aspect et en particulier dans le but de réaliser un vaccin vétérinaire, la présente demande propose des compositions immunogènes caractérisées en ce qu'elle comprennent la toxine AC-Hly de B. bronchiseptica et un extrait bactérien contenant tout ou partie des protéines codées par les gènes vrg de B. bronchiseptica.

Comme indiqué précédemment au sujet de B. pertussis et B. parapertussis, la toxine AC-Hly de B. bronchiseptica est contenue dans un extrait bactérien comprenant tout ou partie des facteurs de virulence (adhésines et/ou toxines) de B. bronchiseptica.

Il s'agit avantageusement d'un extrait urée vag, obtenu par exemple à partir de la souche B. bronchiseptica 973S déposée à la CNCM le 12 Mai 1989 sous le n° I-858.

De façon analogue, l'extrait urée vrg est de préférence obtenu à partir de la souche B. bronchiseptica 973S déposée à la CNCM le 12 Mai 1989 sous le n° I-858, selon les modalités décrites ci-dessus pour B. pertussis.

Selon un autre mode de réalisation particulier de l'invention, une composition immunogène capable d'induire la production d'anticorps contre B. bronchiseptica, telle que décrite ci-dessus, comprend des composants polypeptidiques caractéristiques des flagelles de B. bronchiseptica. Ces composants ont été décrits par Akerley B.J. et al (J. of Bact. Feb 1992, p. 980-990).

L'immunité humorale anti-Bordetelle a longtemps été considérée comme la seule importante. En effet, il est connu depuis très longtemps qu'après infection, on peut détecter des anticorps anti-PTX et anti-FHA dans le sérum des malades. De plus, il est possible de protéger passivement contre la maladie, dans le modèle murin, avec des anticorps anti-PTX. Les anticorps circulants jouent donc un rôle dans la neutralisation de la PTX et dans l'inhibition de l'attachement de la bactérie.

Cependant, il n'a jamais été mis en évidence de corrélation entre le taux d'anticorps spécifiques de ces antigènes dans le sérum des individus vaccinés ou infectés et la protection contre la maladie. Très peu de choses sont connues concernant l'immunité des autres facteurs, en particulier l'AC-Hly, la PRN et le LPS. Il a toutefois été montré

\*    qu'il est possible de protéger passivement des souris contre une infection à B. pertussis avec des anticorps anti-AC-Hly.

\* que dans les sérums d'enfants infectés non vaccinés (dont l'âge est supérieur à 8 mois afin d'éviter la présence d'anticorps maternels qui peuvent fausser l'interprétation de la sérologie, et de moins de deux ans afin de connaître le passé clinique) ou des sérums de souris infectées avec des isolats cliniques de B. pertussis ou de B. bronchiseptica l'on peut détecter des anticorps anti-AC-Hly, très précocément, indiquant que cette protéine est exprimée in vivo et dès le début de l'infection, des anticorps anti-FHA, des anticorps anti-PTX, et des anticorps anti-LPS. Les anticorps anti-PRN apparaissent beaucoup plus tardivement et ne persistent pas très longtemps (Guiso, Grimprel, Anjak et Bégué et 1993, Eur. J. Clin. Microbiol. Infect. Dis. 12:596-600).

La vaccination avec le vaccin cellulaire induit aussi la synthèse d'anticorps contre ces facteurs alors que les vaccins acellulaires testés jusqu'à ce jour n'induisent des anticorps que contre les facteurs qui les constituent.

Diverses études suggèrent en outre que l'immunité cellulaire serait aussi requise pour la protection contre l'infection. Plusieurs équipes ont montré que les Bordetelles peuvent pénétrer dans différents types cellulaires, cellules épithéliales, lymphocytes, moncytes, et y persister, in vitro. Par ailleurs, à la suite d'études épidémiologiques, il a été observé que B. bronchiseptica peut persister chez l'homme très longtemps. Enfin, alors qu'il n'est pas possible d'isoler de bactéries plus de 40 jours après l'infection au niveau pulmonaire chez la souris, il est toujours possible de détecter de l'ADN par PCR. L'ensemble de ces résultats suggèrent que les Bordetelles pourraient persister intracellulairement chez l'hôte à un moment donné de l'infection et que l'immunité cellulaire pourrait être requise pour contrôler un état intracellulaire qui permettrait ainsi aux bactéries d'échapper aux défenses immunitaires de l'hôte.

La démonstration de l'implication des cellules T au cours de l'infection a été obtenue récemment (Redhead, Watkins, Barnard et Mills 1993, Infect. Immun. 61:3190-3198). En effet, à dose sublétale, les souris normales éliminent les bactéries en 30-40 jours alors que les souris déficientes en cellules T (souris "nude") sont incapables d'éliminer les bactéries et développent une infection chronique. Le transfert de cellules T de souris convalescentes chez les souris "nude" rend celles-ci capables d'éliminer les bactéries. De plus, les cellules de la rate de souris convalescentes produisent des taux élevés d'IL-2, IFN-$\gamma$ et TNF, mais pas d'IL-4 et d'IL-5. Ce profil est caractéristique des sous-populations des cellules T que sont les cellules de type Th1. L'immunisation des souris avec le vaccin entier induit aussi une réponse de type Th1 et une réponse anticorps modérée alors que l'immunisation avec un vaccin acellulaire composé de PTX, FHA et PRN induit une réponse de type Th2 et un très haut taux d'anticorps. S'il existe une forte corrélation entre des taux élevés d'IgG sériques spécifiques d'antigènes de B. pertussis et une élimination de la bactérie des poumons, des réponses cellulaires directes sont cependant nécessaires pour une élimination complète de la bactérie. L'élimination des bactéries moins rapide chez les souris immunisées avec des vaccins acellulaires qu'avec des vaccins entiers qui ont été observés serait donc due au fait que le vaccin acellulaire ne favoriserait pas l'induction de cellules Th1. Quelque soit le mécanisme d'action des Th1, il est donc maintenant clair que les cellules T jouent un rôle important non seulement indirect en stimulant la synthèse d'anticorps mais aussi direct dans l'immunité anti-B. pertussis via le recrutement, la stimulation et l'activation de cellules phagocytaires, telles que les macrophages et les polynucléaires neutrophiles.

Il a en outre été observé que les Bordetelles peuvent persister chez l'homme, en particulier B. bronchiseptica. Dans certains cas, la bactérie persiste plusieurs semaines dans d'autres plusieurs mois. Au cours de certaines infections, les isolats ont un aspect différent. Ces aspect différent correspond à l'arrêt soit de la synthèse d'AC-Hly, soit de la synthèse de tous les facteurs codés par les gènes vag.

Les inventeurs ont observé de façon tout à fait intéressante dans le cadre de la recherche d'une protection efficace contre la maladie causée par une infection due à des Bordetelles et contre la persistance chez l'hôte de ces bactéries, que les extraits bactériens selon l'invention provoquent une réponse immunitaire humorale et une réponse immunitaire cellulaire.

Ils ont montré qu'après infection par B. bronchiseptica, il y a induction d'une immunité humorale et d'une immunité cellulaire comme dans le cas d'une infection à B. pertussis. De plus, après vaccination avec de l'AC-Hly purifiée, il y a induction d'une immunité de type humoral et cellulaire semblable à celle induite après infection ou réinfection.

L'invention a aussi pour objet des compositions vaccinantes comprenant, à titre de principe actif, une composition immunogène répondant à l'une des définitions données dans les pages qui précèdent, en association avec un véhicule pharmaceutique acceptable et le cas échéant avec un adjuvant.

Comme les vaccins coquelucheux actuellement disponibles sur le marché, le vaccin selon l'invention peut être associé à d'autres principes actifs vaccinants, par exemple ceux du vaccin contre la diphtérie, la polio ou des maladies à Haemophilus ou de façon générale à tout constituant immunogène, par exemple une toxine ou un agent pathogène inactivé déterminé.

Une composition vaccinante selon l'invention peut être spécifique d'espèce et en conséquence capable d'induire une protection contre B. pertussis ou B. parapertussis ou B. bronchiseptica. Au contraire, il peut s'agir d'un mélange comprenant à titre de principe actif une composition immunogène contre B. pertussis telle que définie précédemment et une composition immunogène contre B. parapertussis.

Selon un autre mode de réalisation de l'invention, la composition vaccinante contient les compositions immunogènes élaborées selon l'invention contre B. pertussis et contre B. parapertussis et contre B. bronchiseptica.

Un vaccin particulièrement préféré contre B. pertussis est caractérisé en ce qu'il comprend à titre de principe actif, un "extrait urée vag" de B. pertussis et un "extrait urée vrg" de B. pertussis, la souche de B. pertussis utilisée pour préparer ces extraits étant de préférence la souche HAV déposée à la CNCM sous le n° I-1485.

un vaccin particulièrement préféré contre B. parapertussis est caractérisé en ce qu'il comprend à titre de principe actif, un "extrait urée vag" de B. parapertussis et un "extrait urée vrg" de B. parapertussis, la souche de B. parapertussis utilisée pour préparer ces extraits étant de préférence la souche n° 1 déposée à la CNCM sous le n° I-1498.

Un vaccin particulièrement préféré contre B. bronchiseptica est caractérisé en ce qu'il comprend à titre de principe actif, un "extrait urée vag" de B. bronchiseptica et un "extrait urée vrg" de B. bronchiseptica, la souche de B. bronchiseptica utilisée pour préparer ces extraits étant de préférence la souche 973S déposée à la CNCM sous le n° I-858.

Toute composition vaccinante comprenant un mélange des extraits urée vag et des extraits urée vrg de B. pertussis et/ou de B. parapertussis et/ou de B. bronchiseptica entre également dans le cadre de l'invention.

Le cas échéant, les extraits urée vag et vrg mis en oeuvre sont préparés à partir de plusieurs isolats des Bordetella utilisés.

L'invention propose également un vaccin comprenant à titre de principe actif, des souches de B. pertussis exprimant les gènes vag et des souches de B. pertussis exprimant les gènes vrg, et/ou des souches de B. parapertussis exprimant les gènes vag et des souches de B. parapertussis exprimant les gènes vrg, et/ou des souches de B. bronchiseptica exprimant les gènes vag et des souches de B. bronchiseptica exprimant les gènes vrg.

L'invention vise aussi les extraits bactériens de type "extraits urée" tels qu'obtenus par mise en oeuvre du procédé décrit en détail dans la partie expérimentale.

Entrent également dans le cadre de l'invention, des compositions immunogènes ou des compositions vaccinantes dans lesquelles l'AC-Hly est présente sous forme pure ou en ce qu'elle est remplacée par un polypeptide comprenant sa partie C-terminale et/ou un polypeptide comprenant sa partie interne.

L'AC-Hly peut être isolée à partir de Bordetella ou préparée par exemple par les techniques du génie génétique.

L'invention a également pour objet des anticorps tels que produits chez un hôte, auquel on a préalablement administré des compositions immunogènes ou vaccinantes telles que décrites ci-dessus ou un extrait bactérien. FIGURES

Figure 1 : Détection d'anticorps sériques spécifiques après immunisation par un extrait urée vag Bordetella bronchiseptica, inactivé ou non.

Figure 2: Lymphoprolifération: Vaccin extrait urée vag Bordetella bronchiseptica.

Figure 3: Infection respiratoire à Bordetella bronchiseptica.

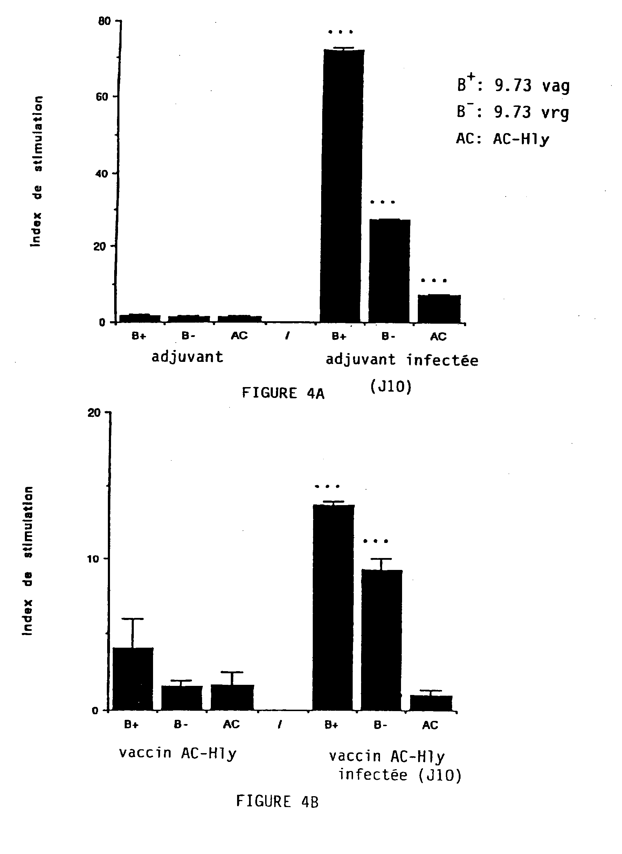

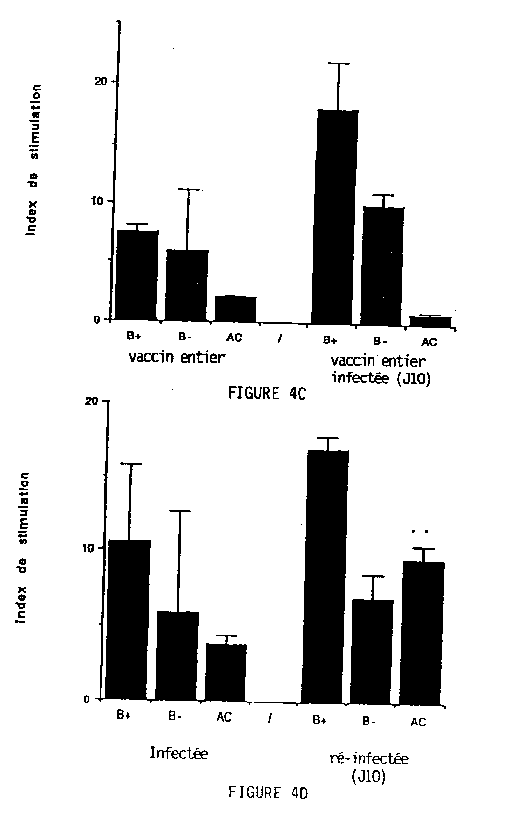

Figure 4: Evolution de l'infection et de la ré-infection à B. bronchiseptica chez des souris primo-infectées avec ces bactéries ou vaccinées avec ces bactéries ou avec de l'AC-Hly purifiée.

Figure 5: Virulence comparée de B. bronchiseptica 973S et d'un mutant B. bronchiseptica aflagellé (973S fla-).

Partie expérimentale

En raison de l'ensemble des résultats exposés ci-dessus, le modèle suivant est proposé pour expliquer le devenir de la Bordetelle chez l'hôte :

Etape 1. Pour initier l'infection, la bactérie exprimerait les gènes vag, les adhésines tels la FHA, les AGG et la PRN permettant ainsi l'adhésion de la bactérie sur les cellules cibles et les toxines telles l'AC-Hly et la PTX permettant ainsi la destruction de la première ligne de défense de l'hôte.

Etape 2. Le but de la bactérie étant de persister et non de tuer l'hôte, elle ne peut continuer à synthétiser de façon continue des toxines comme l'AC-Hly ou la PTX qui détruisent les défenses de l'hôte, en particulier, macrophages et lymphocytes. Elle arrêterait donc la synthèse de ses toxines mais continuerait à synthétiser ses adhésines.

Etape 3. Afin de persister et d'échapper aux défenses immunitaires de l'hôte qui se seraient développées contre les adhésines, la bactérie arrêterait la synthèse des adhésines et exprimerait alors les protéines codées par les gènes vrg. Les arguments que nous avons en faveur de ce modèle sont que:

- des souches de Bordetelles exprimant soit tous les gènes vag soit les gènes vag codant pour les adhésines uniquement soit aucun gène vag sont isolées au cours de la maladie,

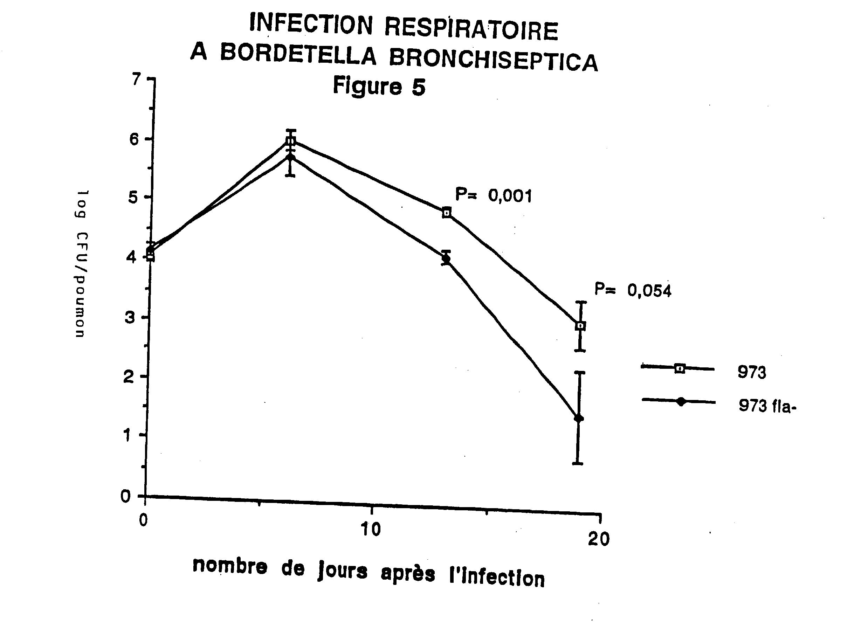

- la capacité d'induire la létalité par les différents isolats cliniques de B. bronchiseptica varie en fonction de la quantité d'AC-Hly exprimée et sécrétée
- après infection de cobayes avec une culture de B. bronchiseptica exprimant les gènes vag, des anticorps dirigés contre les flagelles, produits de gènes vrg, suggérant que la bactérie fait varier la synthèse de ses différents facteurs au cours de l'infection, peuvent être détectés.
- un mutant de B. bronchiseptica exprimant tous les gènes vag mais n'exprimant pas de flagelline (produit vrg, B.J. Akerley, D.M. Monack, A. Falkow, J.H. Miller.J.Bacteriol. 1992. 174, 980-990) et étant donc immobile, construit au laboratoire par mutagénèse insertionnelle, est, d'après nos résultats préliminaires, moins virulent dans le modèle murin d'infection respiratoire : la DL 50 (dose létale 50) de la souche parentale, B. bronchiseptica 973 S, est d'environ $10^7$ bactéries et celle du mutant fla- est d'environ $5.10^7$ bactéries. Par ailleurs, il semble que ce mutant soit éliminé de l'appareil respiratoire plus rapidement que la souche parentale, suggérant l'importance du flagelle pour la persistance de B. bronchiseptica chez l'hôte (Figure 5).

**Vaccin acellulaire efficace contre la maladie et l'infection induites par <u>Bordetella bronchiseptica.</u>**

En accord avec le modèle précédent, le vaccin acellulaire selon l'invention a donc été défini pour protéger non seulement contre l'effet toxique dû aux différents facteurs mais aussi contre la persistance de la bactérie. Ce vaccin pour être efficace est constitué des facteurs nécessaires à la bactérie pour adhérer, se multiplier mais aussi persister extra ou intracellulairement, donc être constitué à la fois des produits des gènes vag mais aussi des gènes vrg.

Pour limiter le coût résultant de la préparation d'un vaccin constitué de tous ces facteurs purifiés, un vaccin acellulaire d'un coût moindre est celui constitué d'un mélange d'"'extraits urée" obtenus à partir de bactéries exprimant les gènes vag et à partir de bactéries exprimant les gènes vrg.

Un "extrait urée" est composé d'un mélange de protéines exprimées à la surface de la bactérie et qui sont séparées de la bactérie après incubation de celle-ci avec de l'urée 5M. L'"extrait urée vag" de B. bronchiseptica contient entre autres protéines l'AC-Hly, la FHA, la PRN et le LPS (endotoxine) et l'"'extrait urée vrg" contient, plusieurs protéines non encore caractérisées, les flagelles et le LPS.

Dans le cas des Bordetelloses animales, le seul agent de la maladie est B. bronchiseptica et le vaccin est constitué d'un mélange d'"'extrait urée vag" et d'"'extrait urée vrg" préparés à partir d'un ou de plusieurs isolats de B. bronchiseptica, si la diversité entre les souches de B. bronchiseptica, mise en évidence grâce à l'utilisation de la technique d'électrophorèse en champ pulsé, se situe au niveau des protéines constituant ces extraits urée. Il sera important de tester la nécessité d'inactiver les protéines constituants ces extraits, en particulier l'AC-Hly. Par ailleurs, il faudra tester la nécessité de conserver le LPS dans ces extraits.

**Vaccin acellulaire efficace contre la maladie et l'infaction induites par <u>Bordetella pertussis</u> et <u>Bordetella parpertussis.</u>**

Dans le cas des Bordetelloses humaines B. pertussis, B. parapertussis et B. bronchiseptica peuvent être les agents de la maladie. Le vaccin pourrait alors être constitué de suspensions bactériennes exprimant les gènes vag et de suspensions bactériennes exprimant les gènes vrg des représentants des trois espèces de Bordetelles (ce vaccin serait alors constitué de six suspensions bactériennes).

Ces suspensions bactériennes pourraient être obtenues à partir de bactéries dont les toxines ont été inactivées par génie génétique afin de diminuer les effets secondaires dus à la vaccination avec des bactéries entières.

1. Préparation d'extrait urée vag et vrg

1.1. Extrait urée vag

Les extraits urée vag sont préparés à partir de bactéries du genre Bordetella (espèces pertussis, parapertussis ou bronchiseptica) phase I, c'est à dire des bactéries exprimant les gènes vag. Les protéines exprimées à partir des gènes vag sont entre autres : l'adényl cyclase-hémolysine (AC-Hly), la toxine de pertussis (PTX) et les agglutinogènes (AGG).

Le protocole de préparation de ces extraits urée est le même pour les 3 espèces bactériennes.

1.2. Extrait urée vrg

Les extraits urée vrg sont préparés à partir des 3 mêmes espèces bactériennes phase IV c'est à dire à partir de bactéries n'exprimant plus aucun gène vag mais exprimant les gènes vrg. Les produits de ces gènes ne sont pas encore bien caractérisés, à l'exception des flagelles de B. bronchiseptica. Les extraits urée vrg se préparent comme les extraits urée vag.

## 2. Résultats

### 2.1. Préparation d'extrait urée vag à partir de B. bronchiseptica 973 phase I

### 2.1.1. Culture de Bordetella bronchiseptica Phase I

- Cultiver les bactéries phase I sur milieu solide Bordet Gengou au sang (BGS ; décrit plus loin) pendant 48 heures de façon à obtenir des colonies isolées hémolytiques.
- Resuspendre ensuite quelques colonies dans du milieu Stainer liquide (décrit plus loin) et étaler 100 μl de cette suspension sur milieu Stainer solide (CSM ; décrit plus loins).
- Après 24-36 heures de cultures à 36°C, resuspendre les bactéries dans du milieu Stainer liquide et ensemencer une fiole de 2 l contenant 200 ml de milieu Stainer liquide de telle sorte que la densité optique à 650 nm soit de 0,05.
- Après ensemencement, cultiver 18-20 heures à 36°C sous agitation douce de façon à obtenir une densité optique à 650 nm de 1-1,2.
- Doser l'activité adényl cyclase de la suspension bactérienne ainsi obtenue sans la diluer (décrit plus loin). Si celle-ci est comprise entre 30 et 100 U/ml, l'extrait urée peut être préparé.

Pour la préparation des extraits urée vrg, des souches de Bordetella phase IV sont utilisées et cultivées de la même façon que les souches phase I, à l'exception du fait que le milieu Stainer dont question ci-dessus, est complété par du sulfate de magnésium 50 mM. Le cas échéant, le sulfate de magnésium est remplacé par d'autres régulateurs, tels que décrits dans la publication de Melton A., Weiss A., Inf. Imm. 1993, 61:807-815.

### 2.1.2. Préparation de l'extrait urée vag

- Centrifuger la suspension bactérienne 30 minutes à 5000 g à 4°C.
- Resuspendre le culot bactérien dans de l'urée 5M préparée dans du tampon PBS (décrit plus loin) à raison d'un volume égal à 5 fois le poids humide du culot bactérien.
- Laisser sous agitation durant 1 heure à 4°C puis centrifuger 40 minutes à 40000 g à 4°C ,
- Conserver le surnageant à -80°C jusqu'à utilisation après avoir vérifié la présence d'adényl cyclase hémolysine, hémagglutinine filamenteuse et pertactine dans l'extrait à l'aide d'anticorps spécifiques de ces facteurs.

### 2.1.3. Inactivation de l'extrait urée vag

- Après élimination de l'urée, par passage sur colonne G25, l'extrait urée vag est dilué en PBS de façon à obtenir une concentration protéique de 300 μg/ml.
- Ajouter goutte à goutte un volume de glutaraldéhyde à 2,5% de façon à obtenir une concentration finale de 0,05%.
- Laisser 2 heures à température ambiante en mélangeant régulièrement.
- Arrêter la réaction par addition de lysine (concentration finale 0,02M).
- Après 2 heures à température ambiante, l'extrait urée est adsorbé sur hydroxyde d'alumine préparé en PBS (1 mg/ml), pendant une nuit à 4°C sous agitation douce. Le vaccin est alors prêt pour l'immunisation de l'animal à raison de 10 à 20 μg/injection (décrit plus loin).

### 2.2. Efficacité protectrice de l'extrait uréee vag B. bronchiseptica dans le modèle murin d'infection respiratoire.

### 2.2.1. Immunisation

- Immuniser deux groupes de souris à 14 jours d'intervalle avec soit 250 μl d'hydroxyde d'alumine à 1 mg/ml soit 250 μl d'extrait urée vag adsorbé sur hydroxyde d'alumine.

2.2.2. Réponse humorale

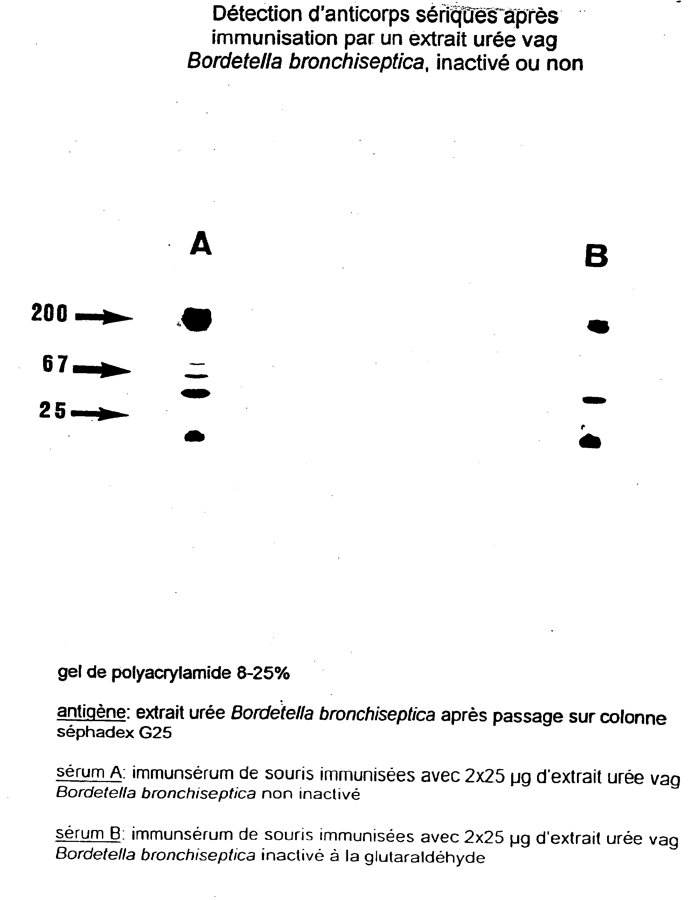

- 7 jours après la 2ème immunisation, vérifier la présence d'anticorps dans le sérum des souris vis à vis des protéines de l'extrait urée <u>vag</u> par la technique de Western Blot (figure 1). Les anticorps produits sont de type IgG incluant des immunoglobulines spécifiques de type IgG2a, de type IgM IgA.

2.2.3. Réponse cellulaire

La réponse induite après immunisation avec la bactérie entière a été comparée à celle obtenue après immunisation avec l'extrait urée vag inactivé ou non ou avec un produit vag purifié, l'AC-Hly.
La réponse cellulaire est étudiée 7 jours après la deuxième immunisation chez une partie des souris.

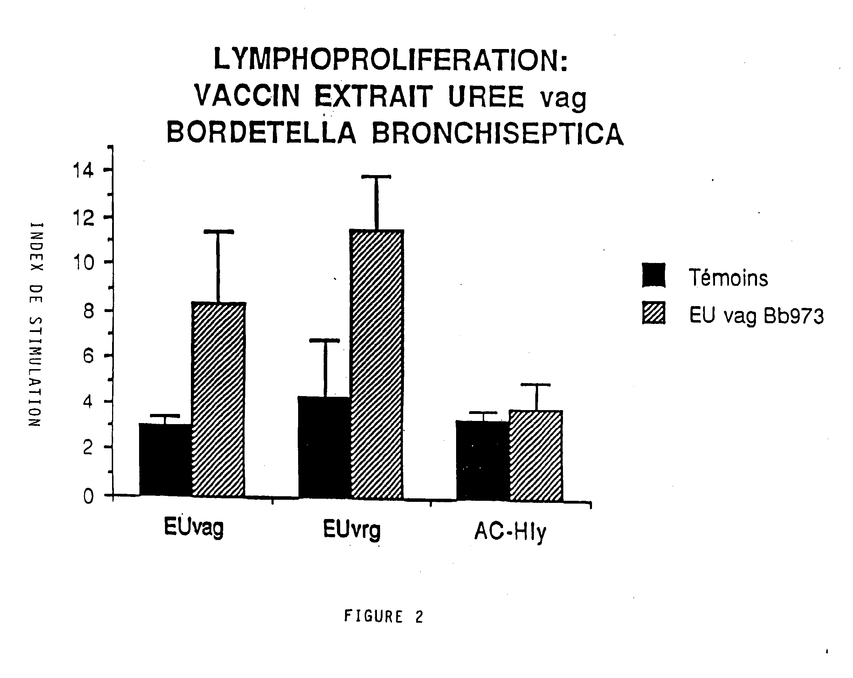

Dans la figure 2, les splénocytes de souris immunisées avec l'extrait urée vag non inactivé ($2x\ 10^6$ cellules par ml) sont mis en culture avec soit l'extrait urée vag (EU vag), soit l'extrait urée vrg (EU vrg), soit l'AC-Hly à une concentration protéique finale de 1 µg/ml (d'après le protocole de Redhead et al).
Une réponse proliférative est obtenue avec l'extrait urée vag et l'extrait urée vrg. Des cytokines caractéristiques de cellules Th1 ou Th2 sont synthétisées.

2.2.4 Epreuve

Le reste des souris est infecté par une dose subléthale de bactéries.

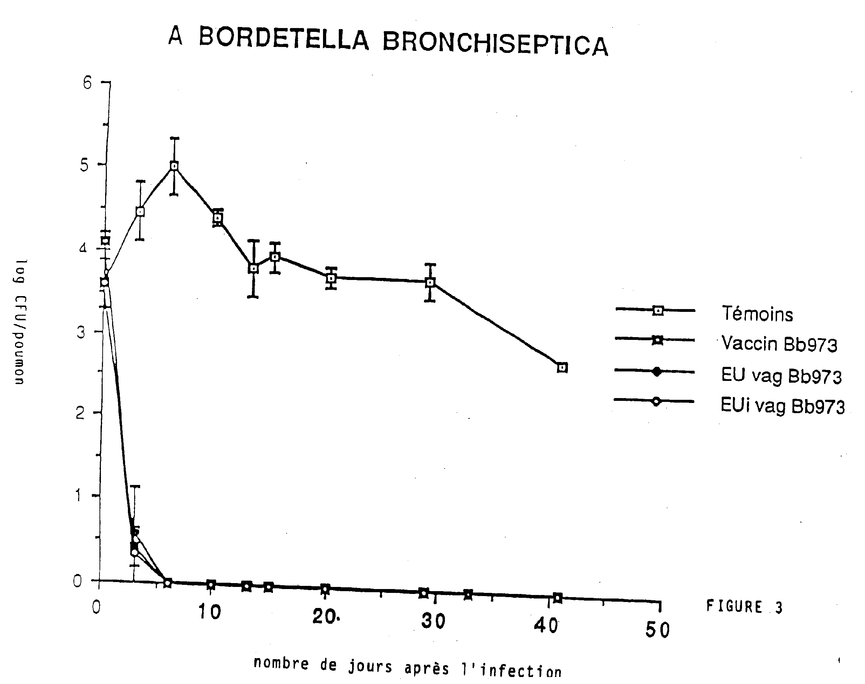

- 14 jours après la 2è immunisation, les 2 groupes de souris sont infectés par voie intranasale avec 50 µl d'une suspension bactérienne de <u>Bordetella bronchiseptica</u> Phase I cultivée en milieu Stainer comme décrit précédemment.
- A $J_0$, $J_3$, $J_6$, $J_{10}$, $J_{12}$, $J_{15}$, $J_{30}$, $J_{40}$, 3 ou 4 souris de chaque groupe sont sacrifiées et une numération des bactéries est faite au niveau du poumon.
- Les résultats obtenus sont semblables à ceux obtenus après vaccination avec un vaccin composé de bactéries entières (figure 3).

Figure 1 : Détection d'anticorps sériques spécifiques après immunisation par un extrait urée vag <u>Bordetella bronchiseptica,</u> inactivé ou non gel de polyacrylamide 8-25%
antigène: extrait urée <u>Bordetella bronchiseptica</u> après passage sur colonne séphadex G25
sérum A: immunsérum de souris immunisées avec 2x25 µg d'extrait urée vag <u>Bordetella bronchiseptica</u> non inactivé
sérum B: immunsérum de souris immunisées avec 2x25 µg d'extrait urée vag <u>Bordetella bronchiseptica</u> inactivé à la glutaraldéhyde.

Description des milieux de culture Milieu Bordet Gengou deshydraté

Composition:

|  | 1 litre | 5 litres |
|---|---|---|
| *Milieu Bordet Gengou* | *30 g* | *150 g* |
| *Glycérol* | *10 ml* | *50 ml* |
| *Eau pyrolysée* | *1 litre* | *5 litres* |
| *Ajuster le pH à 7,4* | | |

- Faire chauffer
- Autoclaver 15 minutes à 120°
- Conserver à 4°C

Au moment de l'emploi :
Le Bordet Gengou est enrichi avec 15 à 20% de sang de mouton ou de cheval. Faire fondre les tubes et les maintenir fondus à 54°C. Rajouter 2,5 ml de sang de mouton dans chaque tube, stérilement. Verser le contenu du tube dans une boîte de pétri stérile.

Remarque:

Pour la recherche de Bordetella pertussis à partir de prélèvement nasopharyngé, utiliser des boîtes fraîches (maximum 7 jours à 4°C)

Milieu Gélose CSM

Pour préparer 2 litres d'une solution concentrée 10 fois :

| Sodium Hydrogénoglutamate | (Réf. Prolabo n°27872.298) | 107g |
|---|---|---|
| L-Proline | (Réf. Merck n°7434) | 2,4g |
| NaCl | (Réf. Prolabo n°27810.295) | 25g |
| H2P04 | (Réf. Prolabo n°26926.298) | 5g |
| KCl | (Réf. Prolabo n°26759.291) | 2g |
| MgCl2 | (Réf. Prolabo n°25108.295) | 1g |
| Tris-base | (Réf. Merck n°8382.2500) | 15,2g |
| Casamino Acids | (Réf. Difco n° 0288-01-2) | 5g |
| Solution de CaCl2 à 1% dans l'eau pyrolysée | (Réf. Prolabo n°22317.297) | 20 ml |
| Eau pyrolysée | QSP | 1 litre |

Dissoudre les différents constituants dans une partie du volume d'eau final. Ajuster le pH à 7,4 à l'aide d'acide chlorydrique. Compléter au volume final et conserver à -20°C.

- Au moment de l'emploi, mélanger:
- 100 ml de la solution concentrée 10 fois
- 900 ml d'eau pyrolysée
- 1 g de (2,6-0-Dimethyl) cyclodextrine référence Aldrich n° 51166-71-3
- 15 g de Bacto agar référence Difco n°0140-01

Répartir par fractions de 20 ml en tube de verre Stériliser et ajouter le complément stérile.

- Solution de complément :
- Mélanger :
- 1 ml de solution de complément concentrée 10 fois
- 100 mg de glutathion référence Merck n° 4090
- 9 ml d'eau pyrolysée

Filtrer cette solution sur filtre millex 0,22 μm
Ajouter 200 μl de cette solution à 1 tube de 20 ml de milieu

Milieu de culture Stainer

A. Milieu de Base

Pour préparer 2 litres d'une solution concentrée 10 fois :

| Sodium Hydrogénoglutamate | (Réf. Prolabo n°27872.298) | 214,0g |
|---|---|---|
| L-Proline | (Réf. Merck n°7434) | 4,8g |
| NaCl | (Réf. Prolabo n°27810.295) | 50,0g |
| H2P04 | (Réf. Prolabo n°26926.298) | 10,0g |
| KCl | (Réf. Prolabo n°26759.291) | 4,0g |

(suite)

| | | |
|---|---|---|
| MgCl2 | (Réf. Prolabo n°25108.295) | 2,0g |
| Tris-base | (Réf. Merck n°8382.2500) | 30,5g |
| Solution de CaC12 à 1% dans l'eau pyrolysée | (Réf. Prolabo n°22317.297) | 40 ml |
| Eau pyrolysée | QSP | 2 litres |

Dissoudre les différents constituants dans une partie du volume d'eau final. Ajuster le pH à 7,6 à l'aide d'acide chlorydrique. Compléter au volume final et répartir cette solution concentrée qui peut être conservée à -20°C pendant plusieurs semaines.
Au moment de l'emploi, diluer le milieu, le stériliser à 120°C pendant 15 minutes puis ajouter le complément stérilisé par filtration.

B. Solution de complément

Pour préparer 200 ml d'une solution concentrée 10 fois :

- L-Cystine (Réf. Prolabo n°23260.184).....8g
- HCl concentré                    20ml

Dissoudre. Sur cette préparation, verser le mélange suivant préalablement dissout :

| | | |
|---|---|---|
| FeS04,7H20 | (Réf. Prolabo n°24244.232) | 2g |
| Acide L(+) ascorbique | (Réf. Prolabo n°20155.237) | 4g |
| Acide nicotinique | (Réf. Merck n°6817) | 0,8g |
| Eau pyrolysée | | 120ml |

Compléter à 200 ml avec de l'eau pyrolysée, répartir la solution par fractions de 1, 2, 3 ou 4 ml et congeler à -20°C.
Au moment de l'emploi, diluer la solution 10 fois dans l'eau pyrolysée et ajouter :

le glutathion (Réf. Merck n°4090)....100mg/10ml de complément dilué, stériliser cette solution par filtration (filtre Millex 0,22 μm à usage unique) et ajouter 1 ml de solution stérile à 100 ml de milieu de base stérile.

Dosage Activité cyclase

Préparation des solutions:

| Mix* | | |
|---|---|---|
| Tris 1M pH8 | 4,15 ml | (83,3 mM final) |
| MgCl2 0,1 M | 5ml | (10 mM final) |
| AMPc 0,34 M | 29μl | (0,2 mM final) |
| eau | 40,5 ml | |
| +200μl AMPc*H$^3$ de façon à avoir $2.14^4$ cpm/60 μl | | |

Après avoir homogénéisé la solution, répartir dans des tubes par 4 ml et conserver à -20°C.

1) Mélange BSA-Calmoduline:

| | |
|---|---|
| BSA 10 mg/ml | 1xμl |

(suite)

| Calmoduline 2µl | 1xµl |
|---|---|
| eau | 2xµl |

La BSA est diluée dans l'eau et la calmoduline est diluée dans du Tris.
ATP*:
ATP froid 20mM
+ATP*P$^{32}$
de façon à avoir environ 8.10$^5$ cpm/10µl (Jour 0)
on peut l'utiliser jusqu'à 5.10$^5$ cpm/10µl

2) Réalisation du dosage:

. Important: faire tous les dosages en double, y compris les témoins.
. Dans chacun des tubes en verre préalablement étiquetés, mélanger:

60µl de Mix*
20 µl de BSA-CaM-eau
10 µl d'échantillon (exemple : suspension bactérienne DO=1, ou diluer avec Tris 50 mM pH8)

. Placer les tubes dans un bain à 30°C puis ajouter 10µl d'ATP* et laisser pendant 10 minutes précisément.
. Ajouter 200µl d'HCl 0,5 M pour arrêter la réaction.
. Plonger les tubes dans un bain marie bouillant pendant 5 minutes puis ajouter 200µl d'imidazole 1,5M. (Etape non nécessaire).
. Laisser refroidir puis déposer sur colonne d'alumine (1 gramme par colonne). Eluer avec 3 ml d'imidazole 10 mM.
. Recueillir l'éluat dans 10 ml de scintillant et compter le nombre de cpm H$^3$ pour calculer le rendement de la colonne et le nombre de cpm P$^{32}$ pour calculer l'activité cyclase. (Attention au "quenching").
. Faire un "blanc": les 10µl d'échantillon sont remplacés par 10µl d'eau.
. Deux "activités totales":

1) H$^3$: 3ml d'imidazole sont élues d'une colonne et recueillis dans des fioles contenant 10 ml de scintillant et 60µl de Mix.
2) P$^{32}$: idem mais les fioles contiennent 10 ml de scintillant et 10µl d'ATP-P$^{32}$.

3) Calculs

1) Calcul du rendement de chaque colonne:

$$\frac{\text{cpm 32P x cpm 3H totaux}}{\text{cpm 3 H de l'échantillon}} = \text{x cpm..... X', X'', X'''...}$$

2) Calcul du blanc:
identique = Y cpm

3) Z= nombre de cpm totaux pour chaque échantilon
X cpm - Ycpm = Z cpm..... Z', Z'', Z'''....

4) cpm P$^{32}$ totaux ---------> 200 nmoles d'ATP
Z cpm ---------> W nmoles

W nmoles pour v µl enzyme
T= temps en minutes

$$N \text{ nmoles/minute/ml} = \frac{W \times \text{facteur de dilution (100 si 10}\mu\text{l}}{10 \text{ (temps)}}$$

Si l'échantillon a été dilué avant le dosage, multiplier par le facteur de dilution.

$$G \text{ nmoles/minute/mg} = \frac{N}{mg}$$

**Préparation de l'ATP et de l'AMPc:**

ATP froid: une fois mis en suspension, utiliser tout de suite ou congeler à -20°C. Ne pas décongeler et congeler plusieurs fois... très fragile! Ne pas conserver longtemps.

Préparer 5 ml d'ATP 20 mM: peser environ 70 mg et ajouter 5 ml de Tris 50 mM pH7. De cet ATP dilué au 1/1000ème, mesurer la D.O. très précisément à 260 nm. Calculer la concentration exacte sachant que:

1 mM ----> $D_{260}$=15,4

ou        $10^{-5}$ M ____> $DO_{260}$=0,154

AMPc froid: Préparer comme l'ATP, il faut seulement dissoudre 32,9 mg/10 ml (on peut chauffer un peu pour dissoudre). De même, déterminer la concentration exacte en mesurant la DO à 260 nm, la solution étant diluée au 1/500ème.

$10^{-5}$ M ----> $DO_{260}$=0,154

Références des produits utilisés

Tris: réf. 8382 chez Merck
BSA, albumine bovine: réf. A-4503 chez Sigma
Calmoduline ou Phosphodiesterase 3':5'-cyclic
nucleotide-activator: réf. P 2277 chez Sigma
Imidazole: réf. I-0152 chez Sigma
Scintillant: Optiphase "HiSafe 3', chez LKB-Pharmacia
ATP froid: réf A-2383 (PM=551,1) chez sigma
ATP $P^{32}$ : Amersham, 500 $\mu$Ci, réf. PB 200 = alpha $P^{32}$
AMPc froid: A-9501 chez Sigma
AMP $H^3$: Amersham [la plus petite dose (10$\mu$Ci)]

**PREPARATION D'UN VACCIN ACELLULAIRE**

Bordetella bronchiseptica
Adsorption sur hydroxyde d'alumine

. Référence hydroxyde d'alumine Al(OH)$_3$: Alhydrogel 3 % Al(OH)$_3$ équivalent à 2% Al$_2$O$_3$ (Superfos Biosector als Denmark)

. Tampon de dilution: tampon phosphate-NaCl 0,15M pH=6,8 Formule (concentré 10 fois): PO$_4$ 0,1M (soit Na$_2$HPO$_4$, $_2$H$_2$O 0,07M
et NaH$_2$ PO$_4$, H$_2$O, 0,03M)
NaCl 1,5M
ph=6,8
diluer 10 fois dans de l'eau pyrolysée au moment de l'utilisation.

. Lavage de l'hydroxyde d'alumine:

5 ml d'Al(OH)$_3$ concentré sont lavés dans 50 ml de tampon x 1 à 2 reprises.

Après le deuxième lavage, les 5 ml d'Al(OH)$_3$ lavés sont repris dans un volume total de 50 ml, soit de l'Al

(OH)$_3$ concentré 2 fois à la finale (concentration finale : 2 mg/ml).

L'adjuvant ainsi préparé est mélangé volume à volume avec la fraction vaccinale et mis à adsorber pendant une nuit à 4°C sous agitation. La concentration finale d'Al(OH)$_3$ est de 1 mg/ml dans le vaccin prêt à être injecté à l'animal.

## Revendications

**1.** Composition immunogène, **caractérisée en ce qu'**elle comprend une protéine adénylcyclase-hémolysine (AC-Hly) ou une partie immunogène de cette AC-Hly, d'une souche de Bordetelle choisie parmi *B. pertussis, B. parapertussis*, ou *B. bronchiseptica*, et **en ce qu'**elle comprend en outre une souche de Bordetelle choisie parmi *B. pertussis, B. parapertussis*, ou *B. bronchiseptica*, dont le gène *bvgS* est muté de façon telle que la bactérie exprime essentiellement les gènes *vrg* au détriment des gènes *vag.*

**2.** Composition immunogène selon la revendication 1, **caractérisée en ce qu'**elle comprend en outre une ou plusieurs adhésines ou toxines de *B. pertussis, B. parapertussis* ou *B. bronchiseptica*, choisie(s) parmi la FHA, les AGG ou la PRN et la PTX.

**3.** Composition immunogène selon la revendication 1 ou la revendication 2, **caractérisée en ce qu'**elle comprend la toxine PTX de *B. pertussis*, la toxine AC-Hly de *B. pertussis* et une souche de *B. pertussis* dont le gène *bvgS* est muté de façon telle que la bactérie exprime essentiellement les gènes *vrg* au détriment des gènes *vag*.

**4.** Composition immunogène selon la revendication 3, **caractérisée en ce que** la toxine AC-Hly de *B. pertussis* est contenue dans un extrait bactérien contenant tout ou partie des facteurs de virulence du groupe des adhésines ou des toxines de *B. pertussis.*

**5.** Composition immunogène selon la revendication 4, **caractérisée en ce que** la toxine AC-Hly de *B. pertussis* est présente sous la forme d'un extrait urée dit "extrait urée vag".

**6.** Composition immunogène selon la revendication 1 ou la revendication 2, **caractérisée en ce qu'**elle comprend la toxine AC-Hly de *B. parapertussis* et une souche de *B. parapertussis* dont le gène *bvgS* est muté de façon telle que la bactérie exprime essentiellement les gènes *vrg* au détriment des gènes *vag.*

**7.** Composition immunogène selon la revendication 6, **caractérisée en ce que** la toxine AC-Hly de *B. parapertussis* est contenue dans un extrait bactérien contenant tout ou partie des facteurs de virulence du groupe des adhésines ou des toxines de *B. parapertussis.*

**8.** Composition immunogène selon la revendication 7, **caractérisée en ce que** la toxine AC-Hly de *B. parapertussis* est présente sous la forme d'un extrait urée dit "extrait urée vag".

**9.** Composition immunogène selon la revendication 1 ou la revendication 2, **caractérisée en ce qu'**elle comprend la toxine AC-Hly de *B. bronchiseptica* et une souche de *B. bronchiseptica* dont le gène *bvgS* est muté de façon telle que la bactérie exprime essentiellement les gènes *vrg* au détriment des gènes *vag*.

**10.** Composition immunogène selon la revendication 9, **caractérisée en ce que** la toxine AC-Hly de *B. bronchiseptica* est contenue dans un extrait bactérien contenant tout ou partie des facteurs de virulence du groupe des adhésines ou des toxines de *B. bronchiseptica.*

**11.** Composition immunogène selon la revendication 10, **caractérisée en ce que** la toxine AC-Hly de *B. bronchiseptica* est présente sous la forme d'un extrait urée dit "extrait urée vag".

**12.** Composition immunogène selon l'une quelconque des revendications 1, 2 ou 9 à 11, **caractérisée en ce qu'**elle comprend en outre des composants polypeptidiques caractéristiques des flagelles de *B. bronchiseptica.*

**13.** Composition immunogène selon la revendication 1 ou la revendication 2, **caractérisée en ce que** la toxine AC-Hly est sous forme pure.

**14.** Composition immunogène selon la revendication 13, **caractérisée en ce que** la toxine est remplacée par des fragments de l'AC-Hly contenant au moins la partie C-terminale de la toxine et/ou au moins un domaine interne de l'AC-Hly.

**15.** Composition immunogène selon l'une quelconque des revendications 1 à 5, **caractérisée en ce que** la souche de *B. pertussis à* partir de laquelle est obtenue la toxine AC-Hly, est la souche HAV déposée à la CNCM sous le n° I-1485.

**16.** Composition immunogène selon la revendication 15, **caractérisée en ce que** la souche de *B. pertussis à* partir de laquelle est obtenue la souche de *B. pertussis* dont le gène *bvgS* est muté de façon telle que la bactérie exprime essentiellement les gènes *vrg* au détriment des gènes *vag*, est la souche HAV déposée à la CNCM sous le n° I-1485.

**17.** Composition immunogène selon l'une quelconque des revendications 1, 2 ou 6 à 8, **caractérisée en ce que** la souche de *B. parapertussis* à partir de laquelle est obtenue la toxine AC-Hly, est la souche n° 1 déposée à la CNCM sous le n° I-1498.

**18.** Composition immunogène selon la revendication 17, **caractérisée en ce que** la souche de *B. parapertussis* à partir de laquelle est obtenue la souche de *B. parapertussis* dont le gène *bvgS* est muté de façon telle que la bactérie exprime essentiellement les gènes *vrg* au détriment des gènes *vag*, est la souche n° 1 déposée à la CNCM sous le n° I-1498.

**19.** Composition immunogène selon l'une quelconque des revendications 1, 2, ou 9 à 12, **caractérisée en ce que** la souche de *B. bronchiseptica* à partir de laquelle est obtenue la toxine AC-Hly, est la souche 973S déposée à la CNCM sous le n° I-858.

**20.** Composition immunogène selon la revendication 19, **caractérisée en ce que** la souche de *B. bronchiseptica* à partir de laquelle est obtenue la souche de *B. bronchiseptica* dont le gène *bvgS* est muté de façon telle que la bactérie exprime essentiellement les gènes *vrg* au détriment des gènes *vag*, est la souche 973S déposée à la CNCM sous le n° I-858.

**21.** Composition vaccinante **caractérisée en ce qu'**elle comprend à titre de principe actif, une composition immunogène selon l'une quelconque des revendications 1 à 20, en association avec un véhicule pharmaceutiquement acceptable et le cas échéant un adjuvant.

**22.** Composition vaccinante selon la revendication 21, **caractérisée en ce qu'**elle comprend, à titre de principe actif, une composition selon l'une quelconque des revendications 3 à 5 et une composition selon l'une quelconque des revendications 6 à 8.

**23.** Composition vaccinante selon la revendication 22, **caractérisée en ce qu'**elle comprend en outre une composition selon l'une quelconque des revendications 9 à 12.

**24.** Composition vaccinante, **caractérisée en ce qu'**elle comprend, à titre de principe actif, des souches de *B. pertussis* exprimant les gènes *vag* et des souches de *B. pertussis* exprimant les gènes *vrg*, et/ou des souches de *B. parapertussis* exprimant les gènes *vag* et des souches de *B. parapertussis* exprimant les gènes *vrg*, et/ou des souches de *B. bronchiseptica* exprimant les gènes *vag* et des souches de *B. bronchiseptica* exprimant les gènes *vrg.*

**25.** Composition vaccinante, **caractérisée en ce qu'**elle comprend, à titre de principe actif, un mélange de souches de *B. pertussis* et/ou *B. parapertussis* et/ou *B. bronchiseptica* exprimant les gènes *vrg*.

**26.** Composition vaccinante selon l'une quelconque des revendications 21 à 25, **caractérisée en ce qu'**elle contient en outre, à titre de principe actif, un autre constituant immunogène, par exemple une toxine ou un agent pathogène inactivé.

Détection d'anticorps sériques après
immunisation par un extrait urée vag
*Bordetella bronchiseptica*, inactivé ou non gel de polyacrylamide 8-25%

antigène: extrait urée *Bordetella bronchiseptica* après passage sur colonne séphadex G25

sérum A: immunsérum de souris immunisées avec 2x25 µg d'extrait urée vag *Bordetella bronchiseptica* non inactivé

sérum B: immunsérum de souris immunisées avec 2x25 µg d'extrait urée vag *Bordetella bronchiseptica* inactivé à la glutaraldéhyde

LYMPHOPROLIFERATION:
VACCIN EXTRAIT UREE vag
BORDETELLA BRONCHISEPTICA

FIGURE 2

A BORDETELLA BRONCHISEPTICA

FIGURE 3

nombre de jours après l'infection

log CFU/poumon

Témoins
Vaccin Bb973
EU vag Bb973
EUi vag Bb973

EP 1 386 616 A1

FIGURE 4A

FIGURE 4B

FIGURE 4C

FIGURE 4D

INFECTION RESPIRATOIRE
A BORDETELLA BRONCHISEPTICA
Figure 5

EP 1 386 616 A1

Office européen
des brevets

# RAPPORT DE RECHERCHE EUROPEENNE

Numéro de la demande

EP 03 01 0630

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| D,X | BEATTIE ET AL: "A VIR-REPRESSED GENE OF BORDETELLA PERTUSSIS IS REQUIRED FOR VIRULENCE" INFECTION AND IMMUNITY, vol. 60, no. 2, pages 571-577, XP002001711 * le document en entier * | 25,26 | A61K39/10 C07K14/235 C12N1/20 C07K16/12 |
| D,A | GUEIRARD ET AL: "VIRULENCE OF BORDETELLA BRONCHISEPTICA:ROLE OF ADENYLATE CYCLASE-HEMOLYSIN" INFECTION AND IMMUNITY, vol. 61, no. 10, pages 4072-4078, XP002001710 * le document en entier,et surtout page 4073,'Materials and Methods' * | | |
| A | KNAPP ET AL: "TWO TRANS-ACTING REGULATORY GENES (VIR AND MOD) CONTROL ANTIGENIC MODULATION IN BORDETELLA PERTUSSIS" JOURNAL OF BACTERIOLOGY, vol. 170, no. 11, pages 5059-5066, XP002001712 * le document en entier * | | |
| D,A | DATABASE MEDLINE 'en ligne! FILE SERVER STN KARLSRUHE; ABRÉGÉ 93123171, BEATTIE ET AL: "REPRESSOR BINDING TO A REGULATORY SITE IN THE DNA CODING SEQUENCE IS SUFFICIENT TO CONFER TRANSCRIPTIONAL REGULATION OF THE VIR-REPRESSED GENES (VRG GENES) IN BORDETELLA PERTUSSIS" XP002001714 & J BACTERIOL, (1993 JAN) 175 (2) 519-27 * abrégé * | | |

DOMAINES TECHNIQUES RECHERCHES (Int.Cl.7)

C07K
A61K

-/--

Le présent rapport a été établi pour toutes les revendications

| Lieu de la recherche | Date d'achèvement de la recherche | Examinateur |
|---|---|---|
| LA HAYE | 8 décembre 2003 | Sitch, W |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
...........
& : membre de la même famille, document correspondant

EPO FORM 1503 03.82 (P04C02)

**EP 1 386 616 A1**

*Office européen*

**des brevets**

**RAPPORT DE RECHERCHE EUROPEENNE**

*Numéro de la demande*

EP 03 01 0630

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | WO 90 13312 A (PASTEUR INSTITUT) 15 novembre 1990 (1990-11-15) * page 7 - page 8; exemple 1 * --- | | |
| A | CHEM. ZENTRALBLATT NR.8-1284,1966 PAGE 2576,ABR G 1292 & KITASATO ARCH.EXP.MED.35,1-11,1962 XP002001713 NAKASE ET AL:'BESTANDTEILE VON BORDETELLA PERTUSSIS.BESONDERS ÜBER DIE SCHUTZSUBSTANZ' * abrégé * ----- | | |
| | | | **DOMAINES TECHNIQUES RECHERCHES (Int.Cl.7)** |

Le présent rapport a été établi pour toutes les revendications

| Lieu de la recherche | Date d'achèvement de la recherche | Examinateur |
|---|---|---|
| LA HAYE | 8 décembre 2003 | Sitch, W |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.....................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 03 01 0630

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-12-2003

| Document brevet cité au rapport de recherche | Date de publication | Membre(s) de la famille de brevet(s) | Date de publication |
|---|---|---|---|
| WO 9013312    A | 15-11-1990 | FR    2646776 A1 | 16-11-1990 |
| | | AT    115861 T | 15-01-1995 |
| | | CA    2033085 A1 | 13-11-1990 |
| | | DE    69015314 D1 | 02-02-1995 |
| | | DE    69015314 T2 | 28-11-1996 |
| | | EP    0424518 A1 | 02-05-1991 |
| | | ES    2066206 T3 | 01-03-1995 |
| | | WO    9013312 A1 | 15-11-1990 |
| | | JP    3506044 T | 26-12-1991 |
| | | JP    3399525 B2 | 21-04-2003 |
| | | PT    94030 A ,B | 08-01-1991 |
| | | US    2002172691 A1 | 21-11-2002 |
| | | US    6214356 B1 | 10-04-2001 |

Pour tout renseignement concernant cette annexe : voir Journal Officiel de l'Office européen des brevets, No.12/82